# EUROPEAN PATENT APPLICATION

(11) **EP 0 628 314 A1**
(43) Date of publication of application: **14.12.1994**
(21) Application number: 94108541.7
(22) Date of filing: 03.06.1994
(51) Int. Cl.: A61K 31/70

(54) **Ophthalmic topical composition containing glucose for inhibiting injury to the corneal endothelium**

(30) Priority: 04.06.1993 JP 134304/93
(71) Applicant: Senju Pharmaceutical Co., Ltd., Osaka-shi, Osaka 541 (JP)
(72) Inventor: Awata, Takashi, Amagasaki-shi (JP); Doi, Koji, Kobe-shi (JP); Nakayama, Hisayuki, Akashi-shi (JP); Sameshima, Shogo, Kobe-shi (JP); Sogo, Shunji, Yao-shi (JP); Matsumoto, Takahiro, Akashi-shi (JP); Yokogaki, Shuichi, Kobe-shi (JP)
(74) Representative: Kinzebach, Werner, Dr.

(57) **Abstract**

A pharmaceutical composition is provided which can be topically applied to the outer ocular area for the purpose of inhibiting injury to the corneal epithelium. The composition may take the form of eye drops or eye ointment which contain certain amount of glucose as an active ingredient. The composition can also contain conventional ingredients of eye drops or eye ointment such as carriers, buffering agents etc. as well as certain preservatives.

## Description

### BACKGROUND OF THE INVENTION

The present invention relates to a pharmaceutical composition for ophthalmic topical administration to the outer ocular area. More specifically, the present invention relates to such a composition usable to inhibit injury to the corneal epithelium.

The cornea is a transparent tissue forming the outer, frontal coat of the eyeball. It allows light from outside to enter the interior of the eyeball, with a certain amount of refraction occurring when the light pass through the cornea. The cornea is composed, from its outermost side, of an epithelial cell layer (epithelium), Bowman's membrane, corneal stroma, Descemet's membrane, and an endothelial cell layer. Because located in the outermost, frontal surface, the epithelium is easily injured by impinging foreign bodies or wearing of a contact lens. In particular, when wearing a contact lens, the epithelium is quite likely to be injured since, in addition to the mechanical stress exerted by the contact lens, there may occur metabolic malfunction caused by oxygen defect due to the lowered oxygen partial tension on the surface of the cornea underlying the contact lens. Metabolic malfunction of the epithelium leads to epithelial edema with increasing number of microcysts and fluorescein-stainable opaque spots, and, eventually, to corneal ulcer.

Therefore, a medicament which can be topically applied to inhibit injury to the corneal epithelium due to foreign bodies or contact lenses would be of use. At present, however, there are no current medicaments shown to have such an effect to a satisfactory degree.

### SUMMARY OF THE INVENTION

Upon this background, the present inventors have found that glucose, when topically applied to the outer ocular area (i.e. the cornea and conjunctiva), inhibits injury to the corneal epithelium.

Therefore, in one aspect, it is the object of the present invention to provide a pharmaceutical composition to be topically applied to the outer ocular area (i.e., to the front surface of the cornea or into the conjunctival sac) of mammals (e.g., humans, rabbits, dogs, cats, bovines, horses, monkeys, etc.) which composition contains glucose as the active ingredient for inhibiting injury to the corneal epithelium. The composition can take the form of eye drops (ophthalmic solutions or suspensions), eye ointment or the like.

In another aspect, it is the object of the present invention to provide a method of inhibiting injury to the corneal epithelium, the method performed by topically administering a glucose-containing composition to the outer ocular area.

In still another aspect, it is the object of the present invention to provide use of glucose for the preparation of a pharmaceutical composition for inhibiting injury to the corneal epithelium due to foreign bodies or wearing a contact lens.

The composition of the present invention can be prepared by mixing glucose with per se known pharmaceutically acceptable carriers, excipients or diluents or the like and may be formulated into ophthalmic topical compositions such as eye drops (ophthalmic solutions or suspensions), eye ointment or the like according to a conventional method.

### DETAILED DISCUSSION

In preparing eye drops of the present invention, glucose, together with various additives such as buffers, pH adjusting agents, isotonizers, thickeners, suspending agents, surfactants, solubilizers, chelating agents and the like, is dissolved in sterile purified water and the pH of the mixture is adjusted to, for example, approximately 3 to about 9, more preferably approximately 4 to about 8.

Eye drops of the present invention contain glucose in the range preferably from 0.00001 to 10.0 w/v% (i.e. from 0.00001 to 10.0 g per 100 ml), more preferably from 0.0025 to 4.0 w/v%, and most preferably from 0.01 to 2.0 w/v%. Such eye drops are topically applied to the eye preferably from 3 to 8 times a day, with one or a few drops in each application.

Eye ointments of the present invention contain glucose in the range preferably from 0.00001 to 10.0 w/w%, more preferably from 0.0025 to 4.0 w/w%, and most preferably from 0.01 to 2.0 w/w%. Such ointments are topically applied to the eye, preferably one to four times a day.

The examples of buffers suitable for eye drops of the present invention include phosphates, boric acid, sodium borate, and salts with organic acids such as acetic acid, citric acid, and the like. The examples of suitable isotonizers include boric acid, salts such as sodium chloride and potassium chloride, and glycerol. The examples of suitable thickeners include hydroxyethylcellulose, hydroxypropylcellulose, methylcellulose, hydroxypropylmethy lcellulose, carboxymethylcellulose and its salt, and the like. The examples of suitable suspending agents include surfactants such as polysorbate 80, and water soluble polymers such as carboxymethylcellulose sodium salt, hydroxypropylmethylcellulose, methylcellulose and polyvinylalcohol. The examples of suitable solubilizers include non-ionic surfactants such as polyoxyethylenehydrog enated castor oil, polyoxyethylene sorbitan monooleate, polyoxyethylene stearate, triglycerides, polyethylene glycols, and α- or β-cyclodextrin and their derivatives, and the like. The examples of suitable pH adjusting agents include alkaline compounds such as sodium hydroxide, borax, and sodium dihydrogen phosphate, or acids such as hydrochloric acid, boric acid, phosphoric acid, acetic acid, and the like. The examples of suitable chelating agents include disodium edetate, sodium citrate, condensed sodium phosphate, and the like.

In general, one or more preservatives are contained in eye drops to prevent microorganisms from growing which may possibly have infiltrated into the container after the first opening of its closure. As the composition of the present invention contains glucose, a nutrition for growth and reproduction of microorganisms, a proper preservative must be employed to provide sufficient inhibition of glucose-assisted growth of microorganisms. Thus, it was necessary to select a proper preservative because not all of the known preservatives for ophthalmic preparations (such as benzalkonium chloride, p-hydroxybenzoates such as methyl p-hydroxybenzoate, propyl p-hydroxybenzoate, benzyl alcohol, phenethyl alcohol, chlorhexidine gluconate, chlorobutanol, TEGO 51, etc.) were expected to be sufficient at acceptable concentrations. The inventors examined the preservative effect of these preservatives and found that only chlorhexidine gluconate or chlorobutanol exhibited excellent preservative effect in the presence of glucose. When chlorhexidine gluconate is employed, its concentration is preferably 0.0001 to 0.1 w/v% , more preferably 0.001 to 0.02 w/v%. When chlorobutanol is employed, its concentration is preferably 0.002 to 2.0 w/v%, more preferably 0.1 to 0.5 w/v%. However, if the concentration of glucose is relatively low and other preservatives (than chlorhexidine gluconate and chlorobutanol) exhibit sufficient preservative effect, any of such other preservatives can also be employed.

If the composition of the present invention is contained in a type of container to be discarded after a extremely short-term use (such as a unit-dose disposable type container), the composition is not required to contain preservatives, since any microbial contamination during application raises no problems because the remaining part of the composition will be discarded with its container following each short-time use.

In preparing eye ointment according to the present invention, glucose is mixed with a conventional eye ointment base by a conventional method. The preferred examples of such base include vaseline, Plastibase (oleaginous ointment base), liquid paraffin, polyethylene glycol, and carboxymethylcellulose.

It is possible that the composition according to the present invention contains other pharmacologically effective compounds, provided that such compounds do not act against the purpose of the present invention of inhibiting injury to the corneal epithelium.

### TEST EXAMPLE 1

Inhibiting effect of glucose on injury to the corneal epithelium due to wearing a contact lens was evaluated. Five male albino rabbits (a - e, mean body weight; 2.9 kg) were selected for the test by measuring their base curves of the cornea and visual inspection so that their base curves may conform to one of the base curves of the contact lenses (7.0, 7.2 and 7.4) to be used in the test and their eyes are assured to be free of any apparent disorders.

Hard contact lenses with corresponding base curves were fitted to both eyes of the animals. Directly afterwards, the animals received 1.0 w/v% glucose aqueous solution (i.e. 1.0 g/100 ml) to the right eye and physiological saline to the left eye as a control, 0.1 ml each. Application of these solutions was repeated total 9 times at one-hour intervals. Ten hours after the start of the test, the contact lenses were taken out and the eyes were inspected after one drop of fluoresceine solution onto the cornea for stained spots on the cornea using a slit-lamp microscope. The findings were compared with the original findings which had likewise been obtained directly before the start of the test. The increase in number and severity of the stained spots between these two inspections (initial and final) was used as the index of injury to the corneal epithelium, and glucose solution was compared with the control using this index. The result is shown in Table 1 below.

**Table 1**

| animals | glucose solution | | physiological saline | |
|---|---|---|---|---|
| | initial | final | initial | final |
| a | - | + 1 | - | + 5 |
| b | - | + 1 | - | + 5 |
| c | - | + 1 | - | + 5 |
| d | - | + 1 | - | + 4 |
| e | - | + 1 | - | + 3 |
| Criterion: - = not stained, +1 = very slight, +2 = slight, +3 = moderate, +4 = somewhat strong, +5 = strong | | | | |

As shown in Table 1, considerable staining was observed in the corneal epithelium of the control group, whereas only very slight staining was noted in the glucose group. This indicates that injury to the corneal epithelium is inhibited by the administration of glucose.

### TEST EXAMPLE 2

The effect of glucose on cultured corneal epithelial cells was evaluated.

Methods: Rabbit corneal epithelial cells (NRCE cells, purchased from KURABO Industries, Ltd.) were plated in each well of a 24-well plate and cultured in a growth medium (RCGM medium). After 80% confluence was reached, the cells were washed with a glucose-free medium (glucose-free Dulbecco modified minimum essential medium) and the growth medium was replaced by the glucose-free medium or one of the media containing glucose at the concentrations of 0.00001, 0.0001, 0.001, 0.01 or 0.1 w/v% which were prepared by the addition of corresponding amount of glucose to the glucose-free medium. After the replacement, the cells were cultured at 37 °C under either a normal atmospheric condition (20 % O₂, 5 % CO₂) or low oxygen condition (2 % O₂, 5 % CO₂). The morphology of the cells were microscopically observed in time, i.e. 0, 2, 4, 6, 8, 10, 12, 20 and 24 hours later.

Results: In the glucose-free medium, the cells showed morphological changes with voids appearing between the cells under either the normal atmospheric condition or the low oxygen conditions since six hours after the replacement, which changes were aggravated with the laps of time. The changes were more serious and quicker in the cells cultivated under the low oxygen condition than in those under the normal condition. In both atmospheric conditions, normal and low oxygen, these changes observed with the cells in the glucose-free medium were found suppressed for the cells cultured in the glucose containing media in a concentration-dependent manner (0.00001 to 0.1 w/v%). This result provides a support to the inhibiting effects of glucose solution on injury to the corneal epithelium observed in test example 1.

### TEST EXAMPLE 3

The effects of various preservatives were evaluated in the eye drops containing 1.0 w/v% glucose. The preservatives tested were chlorhexidine gluconate, benzalkonium chloride, Tego 51, methyl p-hydroxybenzoate plus propyl p-hydroxybenzoate, and chlorobutanol. The formulations tested are shown in Table 2.

**Table 2**

| | A | B | C | D | E |
|---|---|---|---|---|---|
| Glucose | 1.0 g | 1.0 g | 1.0 g | 1.0 g | 1.0 g |
| Aminoethylsulfonic acid | 0.5 | 0.5 | 0.5 | 0.5 | 0.5 |
| NaCl | 0.35 | 0.35 | 0.35 | 0.35 | 0.35 |
| KCl | 0.15 | 0.15 | 0.15 | 0.15 | 0.15 |
| ε-Aminocaproic acid | 0.2 | 0.2 | 0.2 | 0.2 | 0.2 |
| Boric acid | 0.16 | 0.16 | 0.16 | 0.16 | 0.16 |
| Borax | 0.007 | 0.007 | 0.007 | 0.007 | 0.007 |
| EDTA Na | 0.01 | 0.01 | 0.01 | 0.01 | 0.01 |
| Hydroxyethylcellulose | 0.1 | 0.1 | 0.1 | 0.1 | 0.1 |
| Polysorbate 80 | 0.15 | 0.15 | 0.15 | 0.15 | 0.15 |
| Chlorhexidine gluconate | 0.005 | - | - | - | - |
| Benzalkonium chloride | - | 0.005 | - | - | - |
| Tego 51 | - | - | 0.005 | - | - |
| Methyl p-hydroxybenzoate | - | - | - | 0.026 | - |
| Propyl p-hydroxybenzoate | - | - | - | 0.014 | - |
| Chlorobutanol | - | - | - | - | 0.3 |
| Sterile purified water | **total** 100 ml | | | | |

Tested microorganisms were Staphylococcus aureus (ATCC, No. 6538), Escherichia coli (ATCC, No. 8739), Pseudomonas aeruginosa (ATCC, No. 9027), Candida albicans (ATCC, No. 10231), and Aspergillus niger (ATCC, No. 16404).

The procedure for preservative test in U.S. Pharmacopeia was followed, i.e., each of the eye drops with a formulation in Table 2 was inoculated with one of the above microorganisms, and the cell number counted upon the inoculation and then 7, 14, 21 and 28 days after the inoculation. As a result, it was found that only formulation A (chlorhexidine gluconate) and formulation E (chlorobutanol) exhibited a sufficient preservative effect to meet the standard set forth in the U.S. Pharmacopeia.

The following examples are presented as a further disclosure and illustration of the compositions of this invention and are not intended as a limitation thereof.

### EXAMPLE 1 Eye drops (ophthalmic solution)

According to a conventional method, an ophthalmic solution having the following formulation was prepared.

| | |
|---|---|
| Glucose | 0.2 g |
| Sodium chloride | 0.4 g |
| Potassium chloride | 0.15 g |
| ε-aminocaproic acid | 0.2 g |
| Boric acid | 0.3 g |
| Borate | q.s. |
| Disodium edetate | 0.01 g |
| Hydroxyethylcellulose | 0.1 g |
| Chlorhexidine gluconate | 0.005 g |
| Sterile purified water | q.s. |
| Total | 100 ml (pH 6.5) |

### EXAMPLE 2 Eye drops (ophthalmic solution)

According to a conventional method, an ophthalmic solution having the following formulation was prepared.

| | |
|---|---|
| Glucose | 0.5 g |
| Sodium chloride | 0.35g |
| Potassium chloride | 0.15 g |
| ε-aminocaproic acid | 0.2 g |
| Boric acid | 0.3 g |
| Borate | q.s. |
| Disodium edetate | 0.01 g |
| Hydroxyethylcellulose | 0.1 g |
| Chlorhexidine gluconate | 0.005 g |
| Sterile purified water | q.s. |
| Total | 100 ml (pH 6.5) |

### EXAMPLE 3 Eye drops (ophthalmic solution)

According to a conventional method, an ophthalmic solution having the following formulation was prepared.

| | |
|---|---|
| Glucose | 1.0 g |
| Sodium chloride | 0.5 g |
| Potassium chloride | 0.15 g |
| Calcium chloride | 0.015 g |
| Boric acid | 0.05 g |
| Borate | 0.02 g |
| Sodium citrate | 0.2 g |
| Hydroxyethylcellulose | 0.1 g |
| Chlorhexidine gluconate | 0.005 g |
| Sterile purified water | q.s. |
| Total | 100 ml (pH 7.2) |

### EXAMPLE 4 Eye drops (ophthalmic solution)

According to a conventional method, an ophthalmic solution having the following formulation was prepared.

| | |
|---|---|
| Glucose | 0.5 g |
| Sodium chloride | 0.5 g |
| Potassium chloride | 0.15 g |
| Calcium chloride | 0.015 g |
| Boric acid | 0.2 g |
| Borate | 0.05 g |
| Sodium citrate | 0.2 g |
| Hydroxyethylcellulose | 0.1 g |
| Chlorobutanol | 0.005 g |
| Sterile purified water | q.s. |
| Total | 100 ml (pH 7.2) |

### EXAMPLE 5 Eye drops (ophthalmic solution)

According to a conventional method, an ophthalmic solution having the following formulation was prepared.

| | |
|---|---|
| Glucose | 0.2 g |
| Sodium chloride | 0.5 g |
| Potassium chloride | 0.15 g |
| Calcium chloride | 0.015 g |
| Boric acid | 0.3 g |
| Borate | 0.05 g |
| Sodium citrate | 0.2 g |
| Hydroxyethylcellulose | 0.1 g |
| Chlorobutanol | 0.005 g |
| Sterile purified water | q.s. |
| Total | 100 ml (pH 7.2) |

### EXAMPLE 6 Eye drops (ophthalmic solution)

According to a conventional method, an ophthalmic solution having the following formulation was prepared.

| | |
|---|---|
| Glucose | 0.005 g |
| Sodium chloride | 0.5 g |
| Potassium chloride | 0.15 g |
| Calcium chloride | 0.015 g |
| Boric acid | 0.45 g |
| Borate | 0.2 g |
| Sodium citrate | 0.2 g |
| Hydroxyethylcellulose | 0.1 g |
| Benzalkonium chloride | 0.005 g |
| Sterile purified water | q.s. |
| Total | 100 ml (pH 7.2) |

### EXAMPLE 7 Eye ointment

According to a conventional method, an eye ointment having the following formulation was prepared.

| | |
|---|---|
| Glucose | 1.0 g |
| Chlorhexidine gluconate | 0.005 g |
| Liquid paraffin | 1.0 g |
| White vaseline | q.s. |
| Total | 100 g |

## Claims

1. A pharmaceutical composition for ophthalmic topical administration to the outer ocular area for inhibiting injury to the corneal epithelium of a mammal, said composition containing a pharmacologically effective amount of glucose as an active ingredient.

2. The composition of claim 1 which is in a dosage form of eye drops.

3. The composition of claim 1 which is in a dosage form of eye ointment.

4. The composition of claim 1 which further contains chlorhexidine gluconate or chlorobutanol as a preservative.

5. A method of inhibiting injury to the corneal epithelium of a mammal, said method comprising topically administering to the outer ocular area of the mammal a composition containing glucose in a pharmacologically effective amount.

6. The method of claim 5 wherein the composition is in a dosage form of eye drops.

7. The method of claim 5 wherein the composition is in a dosage form of eye ointment.

8. Use of glucose for preparing a composition for ophthalmic topical administration to the outer ocular area for inhibiting injury to the corneal epithelium of a mammal.

9. The use of claim 8 wherein the composition is in a dosage form of eye drops.

10. The use of claim 8 wherein the composition is in a dosage form of eye ointment.
